(19) Europäisches Patentamt
European Patent Office
Office européen des brevets

(11) Publication number: **0 352 850 B1**

# (12) EUROPEAN PATENT SPECIFICATION

(45) Date of publication of patent specification: **19.01.94**

(51) Int. Cl.5: **C07D 301/10**, C07D 303/04

(21) Application number: **89201909.2**

(22) Date of filing: **19.07.89**

## (54) A process for producing ethylene oxide.

(30) Priority: **25.07.88 US 224046**
**25.07.88 US 224514**

(43) Date of publication of application:
**31.01.90 Bulletin 90/05**

(45) Publication of the grant of the patent:
**19.01.94 Bulletin 94/03**

(84) Designated Contracting States:
**BE DE ES FR GB IT NL SE**

(56) References cited:
**EP-A- 0 266 015**
**GB-A- 1 055 147**
**US-A- 2 279 470**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Lauritzen, Ann Marie**
**16810 Gaelic Lane**
**Houston Texas 77084(US)**

EP 0 352 850 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing ethylene oxide by passing a gas comprising ethylene, oxygen and a chlorohydrocarbon moderator over a fixed bed of catalyst comprising silver supported on alumina promoted by an alkali metal and rhenium.

Ethylene oxide is conventionally produced by contacting ethylene and oxygen with a supported silver catalyst in the presence of a chlorohydrocarbon moderator which is present in the gas phase. The moderator is added to the gas feed stream and its function is to increase the selectivity of the reaction (i.e., to increase the number of moles of ethylene oxide produced per 100 moles of ethylene consumed). It is believed that the primary mechanism for increasing the selectivity is an inhibition by the moderator of the side reaction wherein ethylene is combusted with oxygen to form carbon dioxide and water. The catalyst utilized in most current commercial fixed bed ethylene oxide reactors is an alkali metal promoted, supported silver catalyst. One problem with this type of catalyst is that increasing levels of moderator beyond the optimum have a deleterious effect on the activity of the catalyst, i.e., the activity decreases as the moderator level is increased GB-A-1 055 147. A decrease in activity means that the temperature of operation of the catalyst will be increased. This can have adverse effects on the longevity of the catalyst. Silver sintering is believed to be a major cause of activity decline for conventional alkali metal promoted, supported silver catalysts. As the silver particles agglomerate, the silver surface area decreases which lowers the number of active sites available for reaction, causing a loss of activity. Both selectivity and activity decline occur faster at higher operating temperatures. Silver sintering also occurs faster at higher operating temperatures. Thus, when operating with conventional alkali metal promoted, supported silver catalysts, it is desirable to minimize the temperature of operation of the catalyst while at the same time optimizing the selectivity. Hence, moderator levels are maintained sufficiently high to obtain a relatively high selectivity but are kept relatively low in order to minimize the activity decline of the catalyst. As a general rule, when operating with conventional alkali metal promoted, supported silver catalysts, the effective moderator level in the gas passing over the catalyst is maintained substantially constant over the operating period of the catalyst US-A-2 279 470.

The newly developed catalysts, comprising silver supported on alumina, promoted by alkali metal and rhenium have a very high selectivity, in the order of 83 to 86 percent EP-A-0 266 015.

It has been found that when operating with the newly developed commercial catalyst comprising silver, alkali metal promoters, and a rhenium promoter supported on an alumina support, longer catalyst lives are obtained when the chlorohydrocarbon moderator level is increased over the period of operation of the catalyst.

The invention relates to a process for producing ethylene oxide which comprises passing a gas comprising ethylene, oxygen and a chlorohydrocarbon moderator over a fixed bed of catalyst comprising silver supported on alumina promoted by alkali metal and rhenium characterized in that under normal operating conditions the concentration of the chlorohydrocarbon moderator in the gas passing over the catalyst is increased during the operation of the catalyst. This increase in moderator level has a beneficial effect on the longevity, that is, the stability of the catalyst over its operating period.

The invention further relates to the process for producing ethylene oxide wherein during a first period of operation the process is operated at a chlorohydrocarbon moderator level sufficient to provide the maximum selectivity to ethylene oxide and after the activity of the catalyst has declined sufficiently to cause operational problems to thereafter in a second period of operation increase the chlorohydrocarbon moderator level by an amount sufficient to cause a significant decrease in the selectivity of the catalyst to ethylene oxide and a corresponding increase in the activity of the catalyst. The significant decrease in selectivity may be a predetermined percentage e.g. greater than 0.5, greater than 1 or greater than 2 percent. The second adjustment of the moderator level allows the catalyst to be operated for a longer period of time, thereby stretching out the period of time required before change out.

Figure 1 represents the activity loss for a supported silver catalyst promoted by alkali metal, rhenium and sulfate as a function of time for a constant moderator level (Curve A) and at an increasing moderator level (Curve B).

Figure 2 represents chlorohydrocarbon moderator levels as a function of time for an alkali metal, rhenium and sulfate promoted, supported silver catalyst used in a commercial operation operated according to the process of the instant invention.

Figure 3 is a schematic representation of the selectivity of a conventional ethylene oxide catalyst (catalyst B) and a rhenium promoted ethylene oxide catalyst (catalyst A) at a constant oxygen conversion level and given set of reaction conditions as a function of moderator level.

Figure 4 is a schematic drawing of the reactor temperature at a constant oxygen conversion level and given set of reaction conditions as a function of moderator level for both a conventional ethylene oxide catalyst (catalyst B) and a rhenium promoted ethylene oxide catalyst (catalyst A).

Figure 5 is a schematic representation of selectivity at a constant oxygen conversion level and given set of reaction conditions versus time for a rhenium promoted catalyst operated in accordance with the process of the instant invention, and

Figure 6 represents the corresponding coolant temperature at a constant oxygen conversion level and given set of reaction conditions as a function of time for the same catalyst operated in accordance with the process of the instant invention.

In commercial operations, ethylene and oxygen are converted to ethylene oxide with the aid of a catalyst which is contained in a ethylene oxide reactor which typically is a large fixed tube sheet heat exchanger containing several thousand tubes filled with catalyst. Coolant is used on the shell side of the reactor to remove the heat of reaction. The conditions for carrying out the ethylene oxidation reaction in the presence of alkali metal and rhenium promoted, supported silver catalysts broadly comprise those described in the prior art. This applies, for example, to suitable temperatures, pressures, residence times, diluent materials such as nitrogen, carbon dioxide, steam, argon, methane or other saturated hydrocarbons, the desirability of employing recycle operations or applying successive conversions and different reactors to increase the yield of ethylene oxide, and any other special conditions which may be selected in processes for preparing ethylene oxide. Pressures in the range from atmospheric to 35 bar are generally employed. Higher pressures, however, are by no means excluded. Molecular oxygen employed as a reactant can be obtained from conventional sources. Typically the required oxygen is normally supplied from an air separation plant. The suitable oxygen charge may consist of relatively pure oxygen, air or a concentrated oxygen stream comprising oxygen in major amount with lesser amounts of one or more diluents, such as helium, nitrogen, argon, carbon dioxide and/or a lower paraffin such as methane. For purposes of illustration only, the following table shows the range of conditions that are often used in current commercial ethylene oxide reactor units.

TABLE 1

| | |
|---|---|
| *GHSV | 1550 - 10,000 |
| Inlet pressure | 10 - 30 barg |
| Inlet Feed | |
| ethylene | 1 - 40% |
| $O_2$ | 3 - 12% |
| $CO_2$ | 2 - 40% |
| ethane | 0 - 3% |
| Argon and/or methane and/or nitrogen diluent | |
| chlorohydrocarbon moderator | 0.3 - 20 ppmv total |
| Coolant temperature | 180 - 315°C |
| Catalyst temperature | 180 - 325°C |
| $O_2$ conversion level | 10 - 60% |
| EO Production (Work Rate) | 33 - 260 g EO/l catalyst/h |

* Liters of gas at standard temperature and pressure passing over one liter of packed catalyst per hour.

A chlorohydrocarbon moderator is added to the ethylene-, oxygen-containing gas stream passing over the ethylene oxide catalyst. This moderator is typically a $C_1$ to $C_8$ chlorohydrocarbon, that is a compound comprising hydrogen, carbon and chlorine. The chlorohydrocarbon moderator may be optionally substituted with fluorine. More preferably the moderator is a $C_1$ to about $C_4$ chlorohydrocarbon. Even more preferably the moderator is a $C_1$ or $C_2$ chlorohydrocarbon. In the most preferred embodiment the moderator is methyl chloride, ethyl chloride, ethylene dichloride, vinyl chloride or mixtures thereof. Preferred moderators are ethyl chloride, vinyl chloride, and ethylene dichloride. Particularly preferred is ethyl chloride.

The catalysts utilized in the process of the instant invention comprise a catalytically effective amount of silver supported on an alumina support promoted by an alkali metal(s) and further promoted by rhenium. In a preferred embodiment, the alkali metal promoter is a higher alkali metal of potassium, rubidium, cesium or mixtures thereof. In a particularly preferred embodiment, the higher alkali metal promoter is cesium. Cesium in combination with lithium also provides advantages. In a further preferred embodiment, a promoting amount of the rhenium co-promoter selected from sulfur, molybdenum, tungsten, chromium and mixtures thereof is also present on the catalyst. In a particularly preferred embodiment the catalyst comprises silver supported on an alpha alumina and promoted by cesium, lithium and rhenium, and in a more particularly preferred embodiment sulfate is also present on the catalyst as a rhenium co-promoter.

The amount of alkali metal promoter preferably present on the catalyst generally lies between 10 and 3000, preferably between 5 and 2000 and more preferably between 20 and 1500 parts by weight (basis metal) per million parts by weight of total catalyst. The amount of rhenium promoter preferably present on the catalyst generally lies between 0.1 to 10, more preferably between 0.2 to 5 millimoles (basis metal) per kilogram of total catalyst. The rhenium co-promoter, when present, preferably will be present on the catalyst in an amount between 0.1 to 10, more preferably between 0.2 to 5 millimoles (basis metal) per kilogram of total catalyst.

In general terms this process is carried out by first bringing the ethylene oxide reactor up to operating conditions with suitable feed gas passing over the catalyst and providing sufficient chlorohydrocarbon moderator to the gas passing over the the catalyst to provide an optimum selectivity. Care is taken however not to provide excessive chlorohydrocarbon moderator substantially over that needed to provide the optimum selectivity. After the catalyst has "lined-out" and normal operating conditions are reached, the catalyst is then run over a period of time with the chlorohydrocarbon moderator being slowly increased over the run time at an average rate of increase of at least $\frac{1}{2}$% per month during the operation of the catalyst, more preferably at an average rate of increase of at least 1% per month and even more preferably at an average rate of increase of at least 3% per month and yet even more preferably at an average rate of increase of at least 5% per month. After the start-up line- out procedure is complete, the chlorohydrocarbon moderator in the stream passing over the catalyst will typically be in the range of about 1 to about 20-25 ppm on a molar basis of the gas stream. With the use of the term "average rate of increase", it is understood that the increase can be either a smoothly increasing function or a stepwise increasing function. In actual plant practice the latter is more likely the case, with the chlorohydrocarbon moderator level being raised a set amount at convenient intervals of time.

The present invention will now be illustrated by means of the following examples.

## Example 1

In this example a catalyst comprising silver supported on an alpha alumina support and promoted with cesium, lithium, rhenium and sulfate was tested in a pilot plant under the following conditions: 15 barg, GHSV of 3300, 40% oxygen conversion, and a feed gas mixture of 30% ethylene, 8% oxygen, 5% carbon dioxide and balance nitrogen. Sufficient vinyl chloride plus ethyl chloride (50/50 mixture) was provided to maintain a moderator level of 3 ppm, measured as vinyl chloride plus ethyl chloride. Selectivities and activities (coolant temperatures) were measured. At given intervals a series of moderator response curves were measured, that is, the moderator level was varied and selectivities and activities were measured. These results are shown in Table 2 below:

TABLE 2

| Time (Days) | VC/EC PPM | SEL. % | Coolant temp. °C |
|---|---|---|---|
| 1 - 8 | 3.0 | 86.1 | 249.4-250.6 |
| 8 | 3.5<br>3.7<br>4.0 | 85.9<br>----<br>85.6 | 247.8<br>---<br>245.6 |
| 20 | 3.0<br>3.5<br>3.7<br>4.0 | 85.6<br>86.5<br>----<br>85.6 | 254.4<br>250.6<br>---<br>245.6 |
| 30 | 3.0<br>3.5<br>3.7<br>4.0 | 85.7<br>86.4<br>----<br>---- | 255<br>252.2<br>---<br>--- |
| 39 | 3.0<br>3.5<br>3.7<br>4.0 | 85.7<br>86.2<br>86.2<br>85.5 | 256.1<br>253.9<br>253.3<br>248.9 |
| 47 | 3.0<br>3.5<br>3.7<br>4.0 | 86.0<br>----<br>86.6<br>86.5 | 256.7<br>---<br>253.9<br>252.2 |
| 59 | 3.0<br>3.5<br>3.7<br>4.0<br>4.5 | 84.8<br>86.0<br>86.5<br>86.5<br>86.1 | 260.6<br>258.3<br>257.2<br>256.1<br>252.2 |

From the moderator response curves were calculated optimum selectivities and the corresponding coolant temperature increases (activity losses) with time. These activity losses are plotted as Curve B in Figure 1. The activity losses for moderator levels of 3 ppm is plotted as Curve A in Figure 1. As can be seen from Table 2 and Figure 1, maintaining the moderator level at 3 ppm results in a substantially linear loss in activity as a function of time, whereas if the moderator level is increased during this time in order to provide an optimum selectivity, the activity loss is much less than when compared to the constant moderator level.

Example 2

This example illustrates the process of the instant invention in a commercial ethylene oxide process. In this process a catalyst comprising silver supported on an alpha alumina support promoted by cesium, lithium, rhenium, and sulfate was utilized. The feed to the reactor after the catalyst had been started up and lined out was on the average maintained at the following concentrations: 0.2% ethane, 6% argon, 30% ethylene, 8% oxygen, 3.5% carbon dioxide, 50% methane, and 2% nitrogen. Ethyl chloride was fed to the reaction as a moderator.

After the initial line-out was complete the chlorohydrocarbon moderator concentration was about 4 ppm. Figure 2 shows the increase in moderator (approximate) level in ppm as a function of time for this commercial operation.

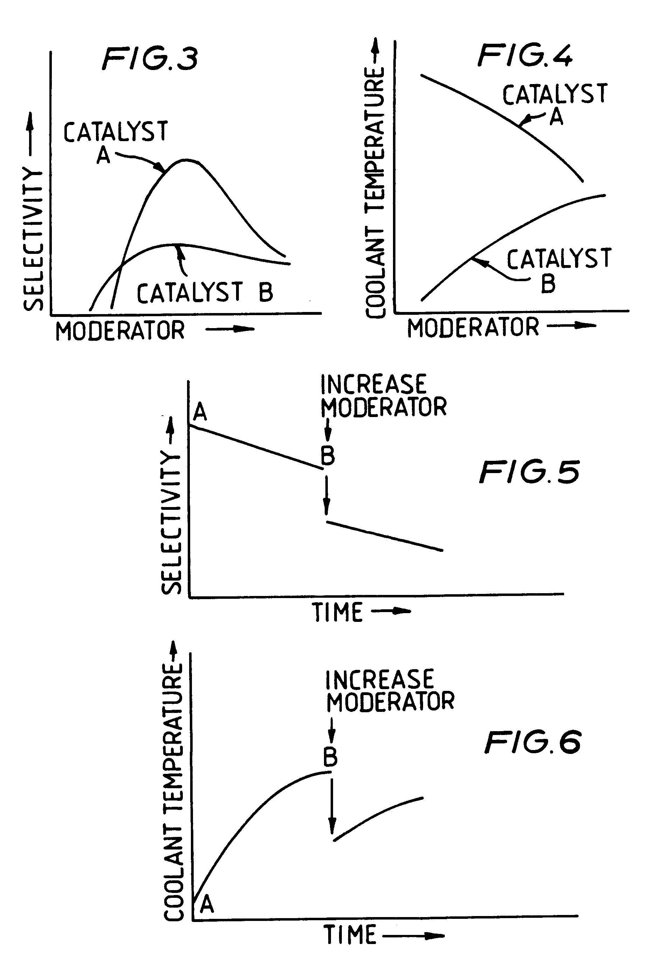

Hereinafter follows a detailed description of the figures 3, 4, 5 and 6:

A series of measurements of selectivity and activity (measured as reactor coolant temperature) were made at a constant oxygen conversion level on a commercial alumina-supported silver ethylene oxide catalyst which was promoted with cesium (catalyst B) and on an alumina-supported silver catalyst promoted with cesium, lithium, rhenium and sulfate (catalyst A) as a function of various levels of a 50:50 mixture of ethyl chloride and vinyl chloride monomer as the moderator. The test conditions were as follows: 3300 gaseous hourly space velocity, 15 barg pressure, 30% ethylene, 8% oxygen, 5% carbon dioxide, balance nitrogen. A generalized summary of these results are presented schematically in Figures 3 and 4. These results show that a sharp selectivity optimum is obtained with the rhenium promoted catalyst and that increasing the moderator level for the rhenium-containing catalyst causes its activity to increase (coolant temperature to decrease).

The selectivity and activity as a function of time for alumina-supported, cesium, lithium, rhenium and sulfate promoted silver catalyst operated according to the process of the instant invention is illustrated in Figures 5 and 6. From the period of time indicated on the drawings from point A to B, the catalyst is operated at moderator levels to provide optimum selectivity. At a certain period of time B the activity of the catalyst has become so low that the coolant temperature has become so elevated that feasible operation of the catalyst is no longer possible. At this point in time, a significant increase in chlorohydrocarbon moderator is made which causes a significant decrease in selectivity of the catalyst with a concomitant increase in activity. This increase in activity allows the catalyst to be operated for a much longer period of time. The trade-off however, is that selectivity has been decreased with the resultant loss in production of ethylene oxide. However, in many cases this is more than balanced by the increased lifetime of the catalyst and the lack of necessity for a quick catalyst change-out.

## Claims

**1.** A process for producing ethylene oxide which comprises passing a gas comprising ethylene, oxygen and a chlorohydrocarbon moderator over a fixed bed of catalyst comprising silver supported on alumina promoted by an alkali metal and rhenium characterized in that under normal operating conditions the concentration of the chlorohydrocarbon moderator in the gas passing over the catalyst is increased during operation of the catalyst.

**2.** A process as claimed in claim 1 wherein the average rate of increase of moderator is at least 0.5 percent per month.

**3.** A process as claimed in claim 1 wherein the average rate of increase of moderator is at least 5 percent, per month.

**4.** A process as claimed in claim 1 wherein during a first period of operation the process is operated at a chlorohydrocarbon moderator level sufficient to provide the maximum selectivity to ethylene oxide and after the activity of the catalyst has declined sufficiently to cause operational problems to thereafter in a second period of operation increase the chlorohydrocarbon moderator level by an amount sufficient to cause a significant decrease in the selectivity of the catalyst to ethylene oxide and a corresponding increase in the activity of the catalyst.

**5.** A process as claimed in claim 4 wherein during the second period of operation the moderator level is increased sufficiently to cause the selectivity to ethylene oxide to decrease by at least 0.5 percent.

**6.** A process as claimed in claim 4 wherein during the second period of operation the moderator level is increased sufficiently to cause the selectivity to ethylene oxide to decrease by at least 2.0 percent.

**7.** A process as claimed in claims 1-6 wherein the catalyst is additionally promoted by a sulfate co-promoter.

**8.** A process as claimed in claim 1 wherein the catalyst is silver supported on alpha alumina promoted by cesium and rhenium.

**9.** A process as claimed in claim 8 wherein the catalyst is silver supported on alpha alumina promoted by cesium, lithium and rhenium.

**10.** A process as claimed in any one of claims 1-9 wherein the moderator is a $C_1$ to $C_8$ chlorohydrocarbon.

**11.** A process as claimed in claim 10 wherein the moderator is a $C_1$ or $C_2$ chlorohydrocarbon.

**12.** A process as claimed in claim 11 wherein the moderator is methyl chloride, ethyl chloride, ethylene dichloride, vinyl chloride or a mixture thereof.

## Patentansprüche

1. Ein Verfahren zum Herstellen von Ethylenoxid, welches das Leiten eines Gases, umfassend Ethylen, Sauerstoff und einen Chlorkohlenwasserstoff-Moderator über ein Katalysatorfestbett, enthaltend Silber auf einem Aluminiumoxidträger, der ein Alkalimetall und Rhenium als Promotoren enthält, umfaßt, dadurch gekennzeichnet, daß bei normalen Betriebsbedingungen die Konzentration des Chlorkohlenwasserstoff-Moderators in dem über den Katalysator geleiteten Gas während des Einsatzes des Katalysators im Betrieb erhöht wird.

2. Ein Verfahren, wie in Anspruch 1 beansprucht, in welchem die durchschnittliche Anstiegsrate des Moderators mindestens 0,5 % pro Monat beträgt.

3. Ein Verfahren, wie in Anspruch 1 beansprucht, in welchem die durchschnittliche Anstiegsrate des Moderators mindestens 5% pro Monat beträgt.

4. Ein Verfahren, wie in Anspruch 1 beansprucht, in welchem während eines ersten zeitlichen Betriebsabschnittes das Verfahren bei einem Gehalt an Chlorkohlenwasserstoff-Moderator durchgeführt wird, der ausreicht, um die maximale Selektivität in bezug auf Ethylenoxid zu erzielen, und in welchem, nachdem die Aktivität des Katalysators soweit herabgesetzt ist, daß Probleme im Betriebsablauf auftreten, während eines zweiten zeitlichen Betriebsabschnittes der Gehalt an Chlorkohlenwasserstoff-Moderator ausreichend erhöht wird, so daß eine wesentliche Abnahme der Selektivität des Katalysators gegenüber Ethylenoxid und ein entsprechender Anstieg der Aktivität des Katalysators erreicht werden.

5. Ein Verfahren, wie in Anspruch 4 beansprucht, in welchem während des zweiten zeitlichen Betriebsabschnittes der Gehalt an Moderator ausreichend erhöht wird, um ein Herabsetzen der Selektivität in bezug auf Ethylenoxid um mindestens 0,5 Prozent zu bewirken.

6. Ein Verfahren, wie in Anspruch 4 beansprucht, in welchem während des zweiten zeitlichen Betriebsabschnittes der Gehalt an Moderator ausreichend erhöht wird, um eine Herabsetzung der Selektivität in bezug auf Ethylenoxid um mindestens 2,0 Prozent zu bewirken.

7. Ein Verfahren, wie in einem der Ansprüche 1 bis 6 beansprucht, in welchem der Katalysator zusätzlich einen Sulfat-Copromotor enthält.

8. Ein Verfahren, wie in Anspruch 1 beansprucht, in welchem der Katalysator ein Silberkatalysator auf einem alpha-Aluminiumoxidträger ist, der Cäsium und Rhenium als Promotoren enthält.

9. Ein Verfahren, wie in Anspruch 8 beansprucht, in welchem der Katalysator ein Silberkatalysator auf einem alpha-Aluminiumoxidträger ist, der Cäsium , Lithium und Rhenium als Promotoren enthält.

10. Ein Verfahren, wie in einem der Ansprüche 1 bis 9 beansprucht, in welchem der Moderator ein $C_1$-$C_8$-Chlorkohlenwasserstoff ist.

11. Ein Verfahren, wie in Anspruch 10 beansprucht, in welchem der Moderator ein $C_1$- oder $C_2$-Chlorkohlenwasserstoff ist.

12. Ein Verfahren, wie in Anspruch 11 beansprucht, in welchem der Moderator Methylchlorid, Ethylchlorid, Ethylendichlorid, Vinylchlorid oder eine Mischung davon ist.

## Revendications

1. Procédé de production d'oxyde d'éthylène, qui consiste à faire passer on gaz comprenant de l'éthylène, de l'oxygène et un modérateur chlorhydrocarboné sur un lit fixe de catalyseur comprenant de l'argent sur un support d'alumine et activé par un métal alcalin et le rhénium, caractérisé en ce que dans des conditions opératoires normales, la concentration du modérateur chlorhydrocarboné dans le gaz passant sur le catalyseur augmente pendant l'opération du catalyseur.

2. Procédé selon la revendication 1, dans lequel le taux moyen d'augmentation du modérateur est d'au moins 0,5% par mois.

3. Procédé selon la revendication 1, dans lequel le taux moyen d'augmentation du modérateur est d'au moins 5% par mois.

4. Procédé selon la revendication 1, dans lequel pendant une première période de fonctionnement, on fait fonctionner le procédé à un niveau de modérateur chlorhydrocarboné suffisant pour procurer le maximum de sélectivité pour l'oxyde d'éthylène et, après la baisse de l'activité du catalyseur à un degré suffisant pour poser des problèmes opératoires, par la suite, dans une seconde période de fonctionnement, on augmente le niveau du modérateur chlorhydrocarboné d'une valeur suffisante pour provoquer une baisse notable de sélectivité du catalyseur pour l'oxyde d'éthylène et une augmentation correspondante de l'activité du catalyseur.

5. Procédé selon la revendication 4, dans lequel pendant la second période de fonctionnement, on augmente suffisamment le niveau du modérateur pour que sa sélectivité à l'oxyde d'éthylène baisse d'au moins 0,5%.

6. Procédé selon la revendication 4, dans lequel pendant la seconde période de fonctionnement, on augmente suffisamment le niveau du modérateur pour provoquer une baisse de la sélectivité à l'oxyde d'éthylène d'au moins 2,0%.

7. Procédé selon les revendications 1 à 6, dans lequel les catalyseur est en outre activé par on sulfate co-activant.

8. Procédé selon la revendication 1, dans lequel le catalyseur est l'argent sur alpha-alumine activé par le césium et le rhénium.

9. Procédé selon la revendication 8, dans lequel le catalyseur est l'argent sur alpha-alumine activé par le césium, le lithium et le rhénium.

10. Procédé selon l'une quelconque des revendications 1 à 9, dans lequel le modérateur est un chlorhydrocarbure en $C_{1-8}$.

11. Procédé selon la revendication 10, dans lequel le modérateur est on chlorhydrocarbure en $C_1$ ou $C_2$.

12. Procédé selon la revendication 11, dans lequel le modérateur est le chlorure de méthyle, le chlorure d'éthyle, le dichlorure d'éthylène, le chlorure de vinyle ou on mélange de ceux-ci.

FIG.1

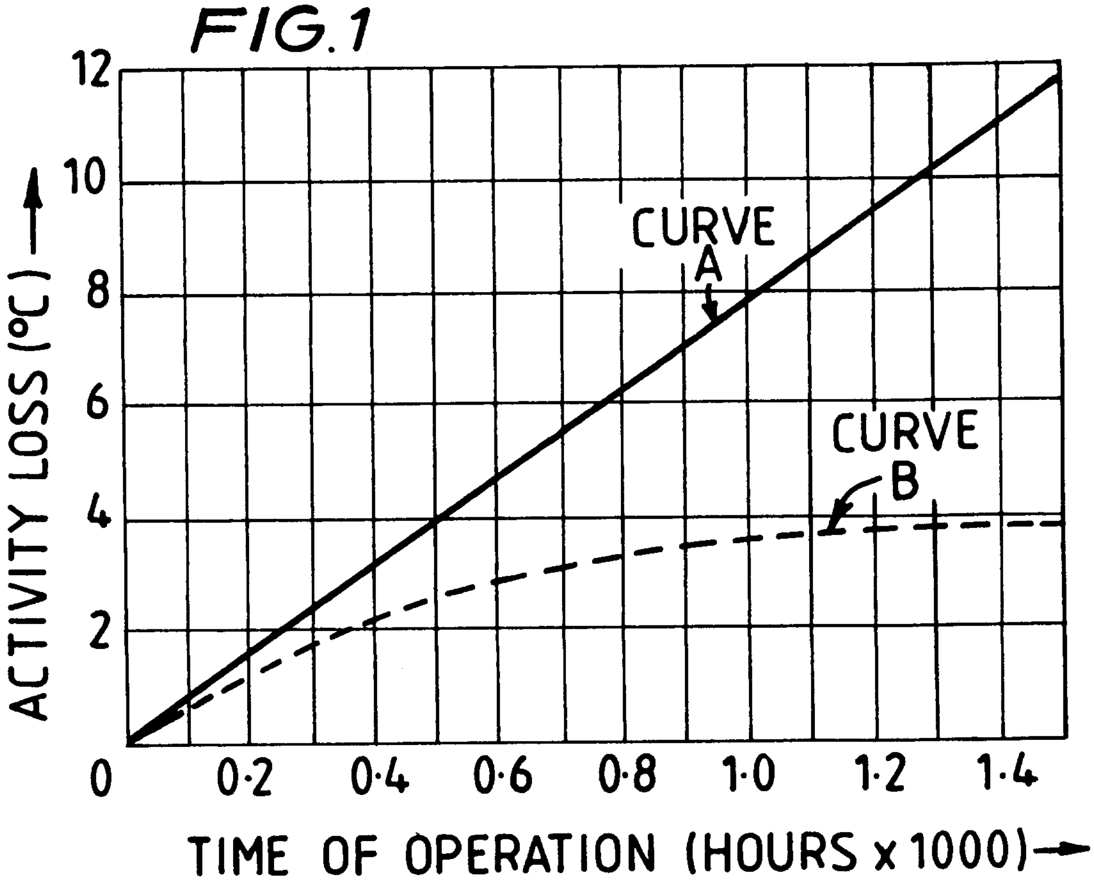
ACTIVITY LOSS (°C)
12
10
8
6
4
2
0
0·2
0·4
0·6
0·8
1·0
1·2
1·4
CURVE A
CURVE B
TIME OF OPERATION (HOURS x 1000)

FIG.2

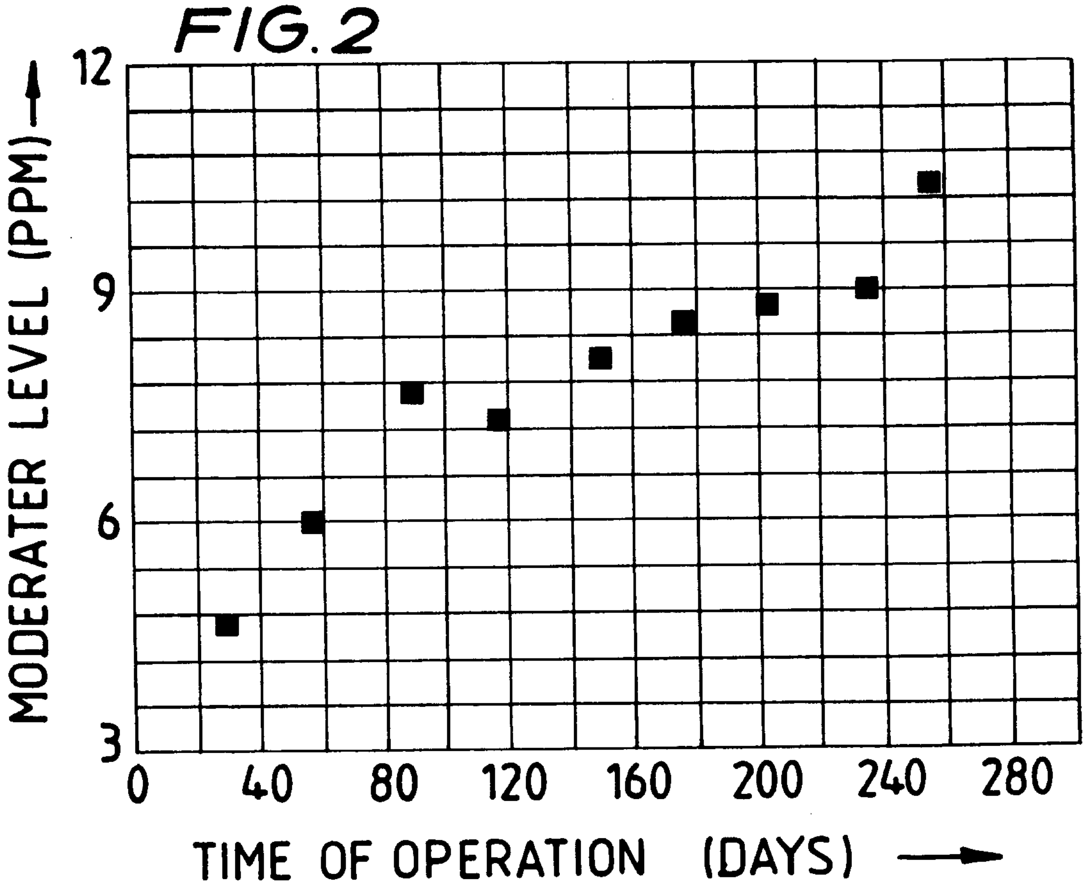
MODERATER LEVEL (PPM)
12
9
6
3
0
40
80
120
160
200
240
280
TIME OF OPERATION (DAYS)

FIG.3

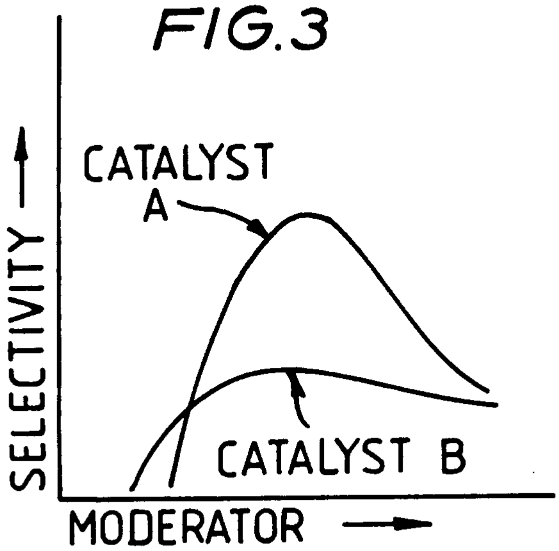


FIG.4

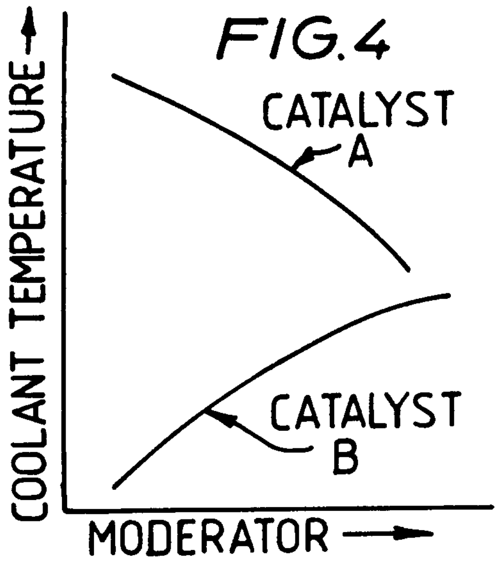


FIG.5

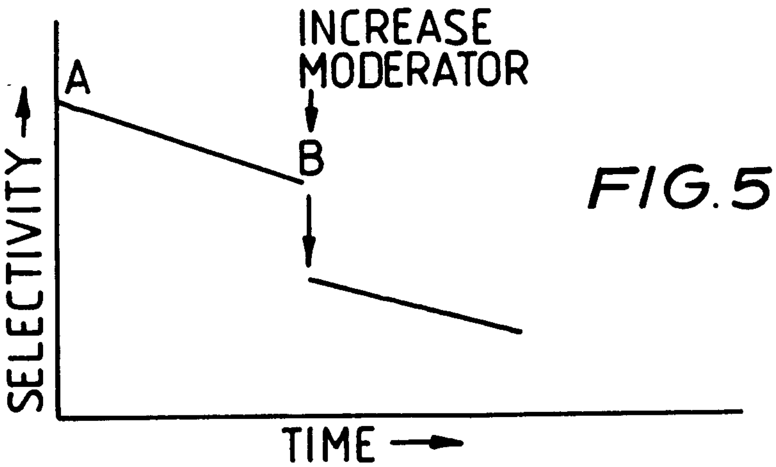


FIG.6